# EUROPEAN PATENT SPECIFICATION

(11) **EP 2 066 247 B1**
(45) Date of publication and mention of the grant of the patent: **14.12.2011**
(21) Application number: 07818461.1
(22) Date of filing: 26.09.2007
(51) Int. Cl.: A61B 17/72, A61B 17/68

(54) **INTRAMEDULLARY OSTEOSYNTHESIS DEVICE**
INTRAMEDULLÄRE OSTEOSYNTHESEVORRICHTUNG
MOYEN D'OSTÉOSYNTHÈSE INTRAMÉDULLAIRE

(30) Priority: 28.09.2006 EP 06425666
(43) Date of publication of application: 10.06.2009
(73) Proprietor: ORTHOFIX S.r.l., 37012 Bussolengo (Verona) (IT)
(72) Inventor: MANTOVANI, Matteo, 37012 Busolengo (Verona) (IT); ROSSI, Luigi, I-37019 Peschiera del Garda (IT)
(74) Representative: Botti, Mario
(86) International application number: PCT/EP2007/008378
(87) International publication number: WO 2008/037454

(56) References cited:
- EP-A1- 0 408 477
- WO-A-99/20195
- WO-A-2005/094706
- DE-U1- 20 213 235
- FR-A1- 2 749 157
- FR-A1- 2 844 701
- US-A- 4 846 162

## Description

### Field of application

The present invention relates in general to the field of osteosynthesis of fractures of a long leg bone, such as for example the femur, or the tibia, in particular an adult's bone.

In a more particular aspect thereof, the present invention relates to an osteosynthesis device of a long bone of a leg, comprising an intramedullary nail adapted for being inserted in a hole formed in the medullary canal of the bone between a bottom and an entrance, and comprising a stiff and elongated body, which is extended along a predetermined axis between a distal end adapted for facing the hole bottom, and an opposite proximal end adapted for facing the hole entrance, a plurality of locking elements inserted in corresponding transversal holes formed in the elongated body of the nail, and an axial stopping element adapted for being constrained to the bone at the entrance of the hole, and constituting an abutment for the proximal end of the nail.

### Prior art

In the field mentioned hereinabove, the need of ensuring the juxtaposition of the fractured portions of a long bone of the leg is well known.

In particular, the fractured bone is perforated within the medullary canal along a predetermined axis for making a hole, called introduction hole, having a diameter slightly larger than the nail. Afterwards, when it is inserted in the hole, the nail is stabilised in the bone by means of locking elements.

Self-tapping screws, or pins, are used as locking elements, which are inserted into corresponding transversal holes formed in the nail body.

In particular, the use of at least two screws is envisaged at a proximal portion of the nail, that is, the portion in the proximity of the introduction point of the nail into the hole, in other words, the hole entrance, and the use of two more screws at a distal portion of the nail, that is, in the proximity of the end, which is opposite to the introduction point, towards the hole bottom.

Despite being advantageous from many standpoints, however the prior art nails exhibit some recognised disadvantages not yet overcome.

The main disadvantage lies in the fact that the known device does not ensure a complete stabilisation of the nail into the leg against the high axial loads the leg is subject to in the direction of the medullary canal, which are determined by the weight of a patient's body.

A destabilisation of the nail occurs in case of application of such loads which causes a destabilisation of the bone fractures, and jeopardizes the osteoshyntesis process.

For this reason, in order to distribute the high axial loads, it is customary to increase the number of locking screws with consequent increase of the perforations in the skin and in the bone, with the disadvantage of an increased risk of infections for a patient.

To avoid the perforations and reduce the invasiveness of the surgical treatment, it has also been proposed to use shape memory elements as locking elements, which are shaped as tabs and distributed on the nail surface. Such a nail is described in patent application WO 2005/094706 in the Applicant's name.

These shape memory elements are mobile between a rest position, taken during the nail insertion in the introduction hole wherein the shape memory elements are retracted on the nail surface, and a locking position wherein they protrude from the nail surface. The locking position is taken by the shape memory elements by the effect of the body temperature, when the nail is inserted in the introduction hole.

The use of shape memory elements, if on the one side solves the disadvantage of perforation by the locking screws, on the other side does not solve the disadvantage mentioned above of the reduced nail stabilisation.

In fact, the shape memory elements in locking position form a relatively small angle with the nail axis, because the protruding locking position is taken by the shape memory elements, when the nail already is in the introduction hole.

As a consequence, the axial load resistance offered by the shape memory elements sometimes is less than that of self-tapping locking screws.

WO -A-99/20195, on which the preamble of claim 1 is based, discloses an intramedullary nail having a elongated body and a plurality of locking elements inserted in corresponding transversal holes formed in the elongated body of the nail. A head element is fixed on the proximal end of the intramedullary nail, and constitutes an abutment for the proximal end of the nail. The head element has at least one bore for receiving at a stable angle bone fixation means.

The head element of WO -A-99/20195 cannot be constrained to the bone without the use of said fixation means. These fixation means have the disadvantage of requiring additional perforations into the bone.

DE 202 13 235 U1 discloses a nail having an axial stopping element adapted for being constrained to the bone at the entrance of the hole, and constituting an abutment for the proximal end of the nail.

However, the axial stopping element of DE 202 13 235 U1 is not able *per se* to ensure a firm stabilisation of the nail into the leg, when excessive axial loads are applied on the nail.The technical problem at the basis of the present invention therefore is to provide an osteosynthesis device of the type mentioned above, which allows the stabilisation of the intramedullary nail into the medullary canal with a reduced number of locking elements and perforations, even when excessive axial loads are applied on the nail.

### Summary of the invention

The technical problem is solved according to the invention by a osteosynthesis device of a leg bone as defined in annexed claim 1.

The main advantage of the present invention lies in the fact of having a combination of transversal locking elements and of a direct stop of the nail into the bone at the proximal end. This combination of elements both ensures a firm stabilisation of the nail and, at the same time, does not require additional perforations.

In fact, the use of the shape memory elements and the use of a stopping element screwed into the hole of the bone exhibits the advantage of ensuring a steady stabilisation of the nail, without perforating the bone for the insertion of additional transversal fixation means.

In addition, thanks to the axial stopping element, the nail is stopped from possible movements which could occur and which would not be prevented by the shape memory elements, due to the low resistance, in the axial direction towards the entrance.

Moreover, the screwing of the stopping element into the hole exhibits the advantage of ensuring a steady fixing of the axial stopping element into the bone.

Moreover, thanks to the screwing of the stopping element, there is the advantage of checking the insertion of the stopping element into the hole entrance till abutment against the proximal end of the nail.

Moreover, since the axial stopping element is constrained to the bone at the hole entrance, it determines a distribution of the axial load in the area comprised between the transversal locking element and the axial stopping element itself. An improved stability and the possibility of reducing the number of locking elements to be used, such as self-tapping screws or shape memory elements, are therefore obtained. In particular the number of locking elements can be reduced at the proximal end of the nail.

According to the invention, the axial stopping element is a body having a substantially cup shape, wherein there are defined a mantle, a bottom, and a free edge, and which form an inner cavity. The proximal end of the nail is housed in the latter.

Preferably, the free edge of the cup element carries front cutting edges for favouring the cup-body advance into the bone.

Even more preferably, the cup body has a very small height, and the inner cavity occupies a prevailing portion thereof, so that the cup body, when is introduced, has relatively small axial overall dimensions that slightly exceed the overall dimensions of the nail at the proximal end thereof.

Thanks to these features, the stopping element is also light, that is, it has a very low weight as compared to the nail so as to little affect the overall weight of the device.

According to the invention, the mantle of the cup body comprises a cylindrical portion occupying a prevailing portion thereof starting from the bottom, and having self-tapping outer threads for obtaining a threaded connection with the bone, and a self-penetrating substantially smooth conical portion, occupying the remaining portion towards the free edge, and capable of progressively enlarging the hole during the introduction into the hole, and preparing the female threads of the hole.

Further features and advantages of the osteosynthesis device according to the invention will appear even more clearly from the following description of a preferred embodiment thereof, made by way of an indicative non-limiting example with reference to the annexed drawings

### Brief description of the figures

Figure 1 shows an osteosynthesis device according to the prior art implanted in a femur.
Figure 2 shows an osteosynthesis device according to the present invention implanted in a femur.
Figure 3 shows a view of another osteosynthesis device according to the present invention implanted in a femur.
Figure 4 shows an enlarged scale view of an axial stopping element of the osteosynthesis device of figures 2 and 3;
Figure 5 shows an enlarged scale section view of a detail of the device of figure 2 in a first operating condition;
Figure 6 shows an enlarged scale section view of a detail of the device of figure 2 in a second operating condition

### Detailed description

With reference to the annexed figures, an osteosynthesis device according to the invention is generally indicated with the reference number 10 and 110.

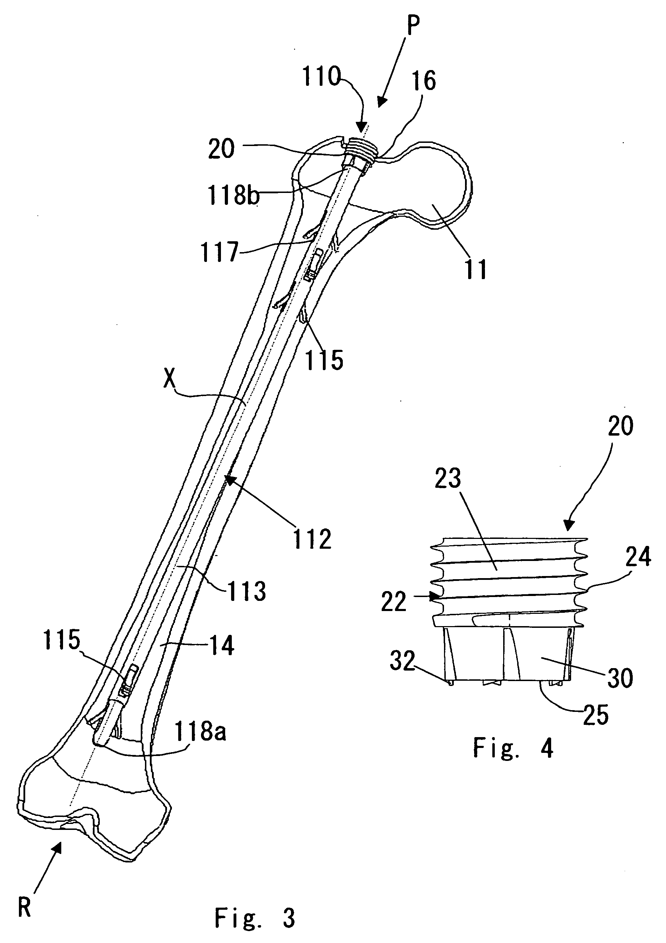

The osteosynthesis device 110 of figure 3 comprises an intramedullary nail 112 inserted in a hole 14, formed in the medullary canal of a long bone 11 between a bottom and an entrance 16, in particular of a femur.

In particular, the osteosynthesis device 110 according to the invention comprises a so-called shape memory intramedullary nail 112, that is, provided with shape memory locking elements 115 distributed on the surface of the nail 112.

The nail 112 comprises an elongated body 113 extended along an axis between a distal tip end 118a and a proximal end 118b, and wherein each shape memory locking element 115 is inserted in a corresponding transversal hole 117 formed in the body 113 of nail 112.

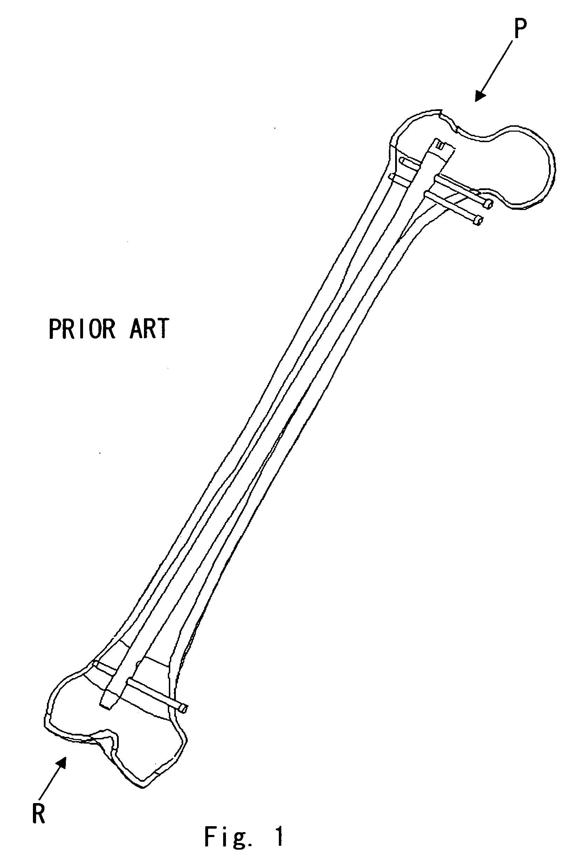

The nail 112 is widely described in the aforementioned international patent application WO 2005/094706 in the Applicant's name. The nail 112 is described in particular in the embodiment illustrated in figures 1 -15d of the above international patent application.

Each shape memory locking element 115 is suitable to take a first shape, or configuration, wherein said elements 115 are retractably housed in the respective hole 117, so as to allow the insertion of the nail in the bone and a second shape or configuration wherein said elements 115 are projecting from the respective hole 17 for allowing the grip and the fixing in the fractured bone.

Shape memory material means a material, already known in the technique, having a given starting shape and taking, under predetermined external conditions or undergoing a predetermined activation condition, after a so-called "material instruction" step, a given new shape.

Within the present invention, the starting shape corresponds to the second shape or configuration wherein the shape-memory elements are arranged projecting form the stem.

Preferably, the new shape, or first shape, is taken by lowering the temperature of the nail.

The particular characteristic of the shape-memory elements stays in that the taking of the second shape or configuration, i.e. the return to the starting shape, under determined physical conditions, continues until the "memorised" starting shape is reached.

This characteristic ensures a constant push, or thrust, into the bone, also in case the bone should be reabsorbed for any reason losing its original shape and size. The expansion temperature of the shape-memory elements from the first to the second shape or configuration can be obtained by means of the body temperature in case a shape-memory material having a so called Af (i.e. end point of the transition while heating), which is lower than, or equal to, 37C° (e.g. 25C°) is used. In case of shape-memory materials having an Af around 48C° suitable heating tools are used.

The main advantage of the shape memory locking elements lies in that they do not require additional perforations into the bone.

Further details about the nail 112 will be described hereafter.

According to the invention, the osteosynthesis device 110 comprises an axial stopping element 20 fixed at the entrance 16 of the hole 14 of the medullary canal and constituting an abutment for the proximal end 118b of the nail 112.

The axial stopping element 20 is an element structurally independent of the nail and is constrained to the bone only, not to the nail, so as to act as a stopping element, or arrest, for the nail 112.

In particular, the axial stopping element 20 is a cup body of titanium, wherein there are defined a cylindrical mantle 22, a bottom 21, and a free edge 25, and which form an inner cavity 35. The proximal end 118b of the nail 112 is housed in the latter.

Even more in particular, the cup body has a very small height L, substantially equal to or slightly larger than, the outer diameter D, in the example L is equal to about 21 mm, and D is equal to about 18 mm.

The inner cavity 35 occupies a prevailing portion of the cup body, and in the example has a height L1 of about 12 mm.

In the illustrated solution, the axial stopping element 20 is screwed in the hole of the medullary canal, so as to prevent additional perforations. According to the invention, the above mantle 22 of the cup body comprises a cylindrical portion 23, occupying a prevailing portion thereof starting from the bottom 21 and carrying self-tapping outer threads 24, and a substantially smooth conical portion 30 (without threads), self-penetrating into the hole 14, occupying the remaining tip portion, towards the free edge 25.

Even more in particular, the free edge 25 carries front cutting edges 32 for further favouring the insertion of the axial stopping element 20, and the forming of the female threads into hole 14.

Preferably, the threads 24 has the outer diameter D of about 18 mm mentioned above, slightly larger than the diameter of the hole 14 for threading the hole 14 during the insertion.

To this end it should be noted that hole 14 is formed in a spongy portion of the bone. For this reason, the outer threads 24 of the axial stopping element 20 has thread, or ridge, pitch and depth, which are suitable for threading the spongy portion of the bone. In particular, the thread has a triangular shape, the pitch is 2 mm, and the depth is 1,5 mm.

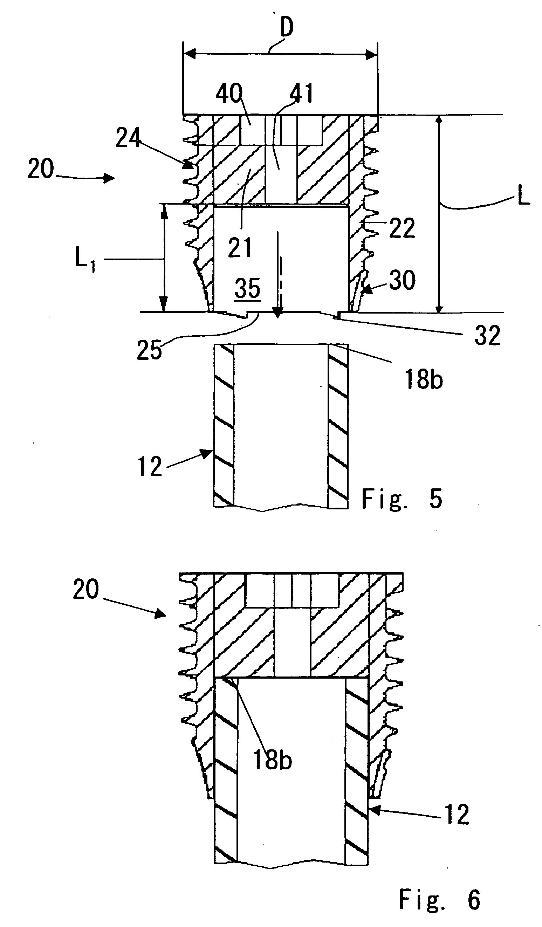

With reference to figure 5, it is further noted that the cup body is completed by a hexagonal recess 40 for being screwed into the bone by means of a special wrench, and a through hole 41 for housing a guide wire.

With reference to the nail 112, in the illustrated embodiment, the shape memory locking elements 115 are inserts structurally independent from the elongated body 113 and have a fork-like shape. In the case of the shown solution, the distal portion of the nail comprises two elements 115, whereas the proximal portion comprises only three elements 115.

As it is illustrated in figure 3, the shape memory locking elements 115 are arranged as offset with one another along the elongated body 113, for example they are arranged with a offset of sexagesimal 90°. The offset arrangement ensures a determined stability on orthogonal planes.

Preferably, as it is possible to see from the figure 3, the elongated body 113 comprises a inner stem and a sheathing tubular jacket wherein the inner stem is inserted. Both the inner stem and the jacket have transversal holes, which form said transversal holes 117. The jacket has the function of retaining, according to the need, the shape-memory locking elements 115 in the first shape, i.e. in the closed retractable position in the transversal holes 117.

In fact, the jacket and the inner stem can be shifted with respect to each other between a first operative position during which the side wall of the jacket closes at least partially the shape-memory locking elements 115 in the first retractable shape in the holes 117, and a second operative position (illustrated in figure 3) wherein the transversal elongate holes of the jacket are aligned with the holes of the inner stem, so as to allow the taking of the second shape by the locking elements 115, and thus their widening apart outside the holes 117.

According to this embodiment of figure 3, both the proximal end of the inner stem and the proximal end of the jacket are housed in the inner cavity 35 of the axial stopping element 20. Therefore, the axial stopping element 20 advantageously enables to prevent the inner stem from shifting with respect to the jacket 46, when the nail 112 is inserted into the bone.

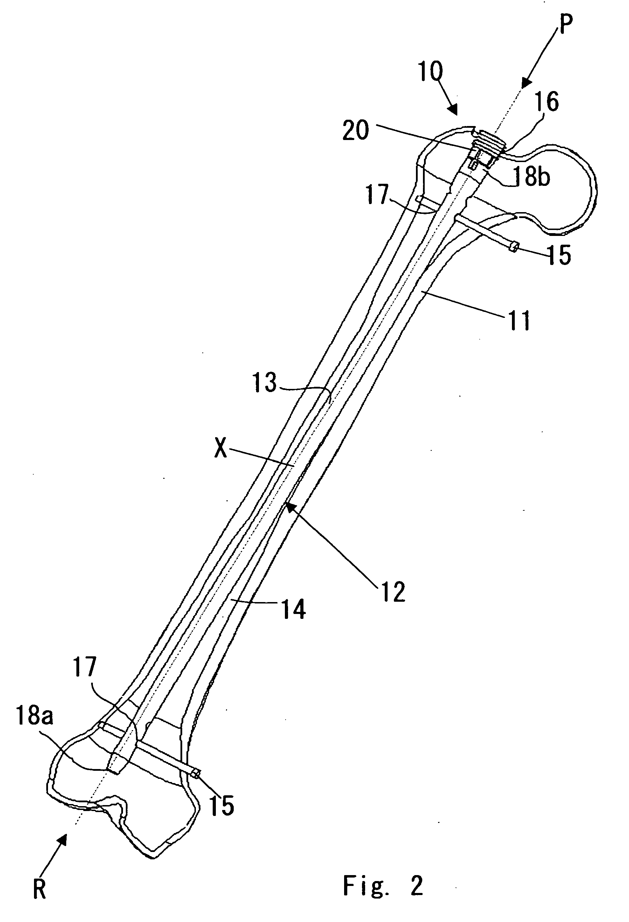

With reference to figure 2 it is disclosed a device 10 having an intramedullary nail 12 made of titanium, comprising an elongated stiff body 13, or stem, extended along a predetermined axis X between a distal end 18a, i.e., facing the bottom of the hole 14 and an opposite proximal end 18b, i.e., facing the entrance 16 of the hole 14. The hole 14 has a substantially cylindrical shape of a size depending on the dimensions of the intramedullary nail 12, which has a diameter preferably comprised between 7 and 13 mm.

The intramedullary nail 12 further comprises locking screws 15 intended for locking the nail 12, and in particular, as it will be better detailed hereinafter, a locking screw 15 in the proximal zone and a locking screw 15 in the distal zone.

Each screw 15 is inserted in a respective transversal hole 17 formed in the body 13.

The osteosynthesis device 10, like the previous one, comprises an axial stopping element 20, having the same cup body shape and the same features as the axial stopping element 20 described above, and arranged as an abutment against the proximal end 18b of the nail 112.

Also in the solution of figure 2, the proximal end 18b of the nail 12 is inserted in a guided manner in the inner cavity 35 of the axial stopping element 20.

With reference to figures 1, 2 and 3, the operation of device 10 when nail 12, 112 is inserted in the medullary canal shall now be described, and this operation is compared with that of a known device.

In particular, reference letters P and R respectively indicate the directions of the axial loads the femur is subject to, that is, the weight force of a patient, which mainly acts on the head of femur 11, and the reaction force exerted by a femur support surface and acting mainly along the medullary canal of the bone, in the direction opposite to the weight.

During the operation of the known device of figure 1, the nail 12, 112 subject to both forces P and R, tends to move into the hole 14 towards the entrance 16, and is therefore destabilised.

In the device 10 of figure 2 or figure 3 according to the invention, the nail 12, 112 subject to the same both forces P, R is kept in correct position by the stop, or abutment, exerted by the axial stopping element 20, which as said above is fixedly constrained to the bone at the proximal end 16.

In the practice, the main advantage of the osteosynthesis device according to the present invention lies in a direct locking at the proximal end of the nail into the bone, with the possibility of reducing the locking elements, in particular those that are the closest to the proximal end, without making additional perforations.

In fact, the axial stopping element 20 and the shape memory locking elements 115 do not require additional perforations into the bone.

In addition, thanks to the axial stopping element 20, the nail 112 is stopped from possible movements which could occur and which would not be prevented by the shape memory elements 115, due to the low resistance, in the axial direction towards the entrance 16. In other words, the axial stopping element 20 compensates the low resistance offered by the shape memory locking elements 115. A firm stabilisation is therefore obtained.

Moreover, another advantage of the device according to the invention lies in the fact that the nail stabilisation is obtained exactly in the proximal zone. In fact, the axial stopping element is close to the proximal end, and therefore the axial load is distributed mainly between the proximal screw and the axial stopping element.

In the solution of figure 3, the improved stabilisation in the proximal zone allows a reduction of the number of shape memory elements 115 of the nail 112 used in this zone, to the advantage of a reduction of the nail manufacturing cost.

In the solution of figure 2, it is further possible to reduce the number of locking screws 15 in the proximal zone from two to one as illustrated in figure 2, so as to reduce the proximal overall dimensions of the screws in this zone, as well as the number of perforations required.
In other words, it is possible to use a single locking screw in the proximal zone.

A further advantage of the present invention lies in moderate overall dimensions and low weight of the axial stopping element, thanks to the small height of the axial stopping element and to the fact that the inner cavity occupies a prevailing portion thereof.

The height of the axial stopping element and the presence of the inner cavity further allow checking the screwing within axis into the introduction hole and obtaining a guided insertion of the proximal end of the nail.

The inner cavity of the axial stopping element has also the advantage of ensuring a steady reciprocal positioning of the axial stopping element and of the nail, when the device is inserted in the bone avoiding side parasitic movements of the proximal portion of the nail.

The axial stopping element also exhibits the advantage of protecting the nail from the fibrous absorption caused by the periosteum at the proximal end, which makes the removal of the nail itself difficult.

The reduction of the overall dimensions of the locking screws has also the advantage of increasing the number and type of fractures that can be treated with the device according to the invention, allowing the treatment of high femur and subtronchanteric fractures.

It is clear that a man skilled in the art may make several changes and variations to the device described above in order to meet specific and incidental needs, all falling within the scope of protection of the invention as defined by the following claims.

## Claims

1. Osteosynthesis device of a long bone (11) of a leg, comprising an intramedullary nail (12, 112) adapted for being inserted in a hole (14) formed in the medullary canal of the bone (11) between a bottom and an entrance (16), and comprising a stiff and elongated body (13, 113), which is extended along a predetermined axis (X) between a distal end (18a, 118a) adapted for facing the bottom of the hole (14), and an opposite proximal end (18b, 118b) adapted for facing the entrance (16) of the hole (14), a plurality of locking elements (15, 115) inserted in corresponding transversal holes (17, 117) formed in the elongated body (13, 113) of the nail (12, 112), and an axial stopping element (20) adapted for being constrained to the bone at the entrance (16) of the hole (14) and constituting an abutment for the proximal end (18b, 118b) of the nail (12, 112); said locking elements being shape memory elements (115), **characterised in that** the axial stopping element (20) is a body having a substantially cup shape, which is defined by a mantle (22), a bottom (21) forming a stopping abutment for the nail (12), and a free edge (25), which form an inner cavity (35), wherein the mantle (22) of the cup body carries an outer threads (24) suitable for being screwed into the bone; the mantle (22) of the cup body comprising a cylindrical portion (23), occupying a prevailing portion of the cup body starting from the bottom (21) and carrying said outer threads (24) and a smooth conical portion (30) with self-penetrating function into the bone, occupying a remaining portion towards the free edge (25) of the cup body.

2. Osteosynthesis device according to claim 1, **characterised in that** the cup body has predetermined height (L) and outer diameter (D), wherein the height (L) has a value substantially equal to or larger than the outer diameter (D).

3. Osteosynthesis device according to claim 1, **characterised in that** the inner cavity (35) occupies a prevailing portion of the cup body.

4. Osteosynthesis device according to claim 1, **characterised in that** the axial stopping element (20) comprises a through hole (41) for housing a guide wire.

5. Osteosynthesis device according to claim 1, **characterised in that** it comprises three locking elements (115) in a portion of the nail facing the proximal end (118b).

6. Osteosynthesis device according to claim 1, **characterised in that** the shape memory locking elements (115) are inserts structurally independent from the elongated body (113).

7. Osteosynthesis device according to claim 1, **characterised in that** the shape memory locking elements (115) have a fork-like shape.

8. Osteosynthesis device according to claim 1, **characterised in that** the shape memory locking elements (115) are arranged as offset with one another along the elongated body (113).

9. Osteosynthesis device according to claim 1, **characterised in that** the elongated body (113) comprises an inner stem and a sheathing tubular jacket, wherein the stem is inserted, and that both the proximal end of the inner stem and the proximal end of the jacket are housed in the inner cavity (35) of the axial stopping element (20).

## Patentansprüche

1. Osteosynthesevorrichtung eines langen Knochens (11) eines Beins umfassend einen intramedullären Nagel (12, 112), der geeignet ist, um in einen Hohlraum (14) eingebracht zu werden, der zwischen einem Grund und einem Eingang (16) in dem medullären Kanal des Knochens (11) ausgebildet ist, und umfassend einen starren und gestreckten Körper (13, 113), der sich entlang einer vorbestimmten Achse (X) zwischen einem distalen Ende (18a, 118a), das dazu geeignet ist, sich dem Grund des Hohlraums (14) zuzuwenden, und einem gegenüberliegenden proximalen Ende (18b, 118b), das dazu geeignet ist, sich dem Eingang (16) des Hohlraums (14) zuzuwenden, erstreckt, eine Vielzahl von Sicherungselementen (15, 115), die in entsprechende querverlaufende Bohrungen (17, 117) eingebracht sind, die in dem gestreckten Körper (13, 113) des Nagels (12, 112) ausgebildet sind, und ein axiales Sperrelement (20), das geeignet ist, um an dem Eingang (16) des Hohlraums (14) an den Knochen festgelegt zu werden und ein Widerlager für das proximale Ende (18b, 118b) des Nagels (12, 112) zu bilden, wobei die Sicherungselemente Formgedächtniselemente (115) sind, **dadurch gekennzeichnet, dass** das axiale Sperrelement (20) ein Körper ist, der im Wesentlichen eine Becherform aufweist, die definiert ist durch einen Mantel (22), einen ein Sperrwiderlager für den Nagel (12) bildenden Grund (21) und einen freien Rand (25), die eine innere Aussparung (35) bilden, wobei der Mantel (22) des Becherkörpers ein zum Eindrehen in den Knochen geeignetes Außengewinde (24) trägt und der Mantel (22) des Becherkörpers einen zylinderförmigen Teilbereich (23), der einen Großteil des Becherkörpers einnimmt, der über am Grund (21) beginnt und das Außengewinde (24) trägt, sowie einen leicht konischen Teilbereich (30) mit selbst in den Knochen eindringender Funktion, der einen Restteil in Richtung des freien Rands (25) des Becherkörpers einnimmt, aufweist.

2. Osteosynthesevorrichtung nach Anspruch 1, **dadurch gekennzeichnet, dass** der Becherkörper eine vorbestimmte Höhe (L) und einen Außendurchmesser (D) aufweist, wobei die Höhe (L) einen im Wesentlichen gleichen oder größeren Wert als der Außendurchmesser (D) aufweist.

3. Osteosynthesevorrichtung nach Anspruch 1, **dadurch gekennzeichnet, dass** die innere Aussparung (35) einen Großteil des Becherkörpers einnimmt.

4. Osteosynthesevorrichtung nach Anspruch 1, **dadurch gekennzeichnet, dass** das axiale Sperrelement (20) eine Durchgangsbohrung (41) zur Unterbringung eines Führungsdrahtes umfasst.

5. Osteosynthesevorrichtung nach Anspruch 1, **dadurch gekennzeichnet, dass** es drei Sicherungselemente (115) in einem dem proximalen Ende (118b) zugewandten Teilbereich umfasst.

6. Osteosynthesevorrichtung nach Anspruch 1, **dadurch gekennzeichnet, dass** die Formgedächtniselemente (115) von dem gestreckten Körper (113) strukturell unabhängige Einlegeteile sind.

7. Osteosynthesevorrichtung nach Anspruch 1, **dadurch gekennzeichnet, dass** die Formgedächtniselemente (115) eine gabelähnliche Form aufweisen.

8. Osteosynthesevorrichtung nach Anspruch 1, **dadurch gekennzeichnet, dass** die Formgedächtniselemente (115) zueinander versetzt entlang des gestreckten Körpers (113) angeordnet sind.

9. Osteosynthesevorrichtung nach Anspruch 1, **dadurch gekennzeichnet, dass** der gestreckte Körper (113) einen inneren Bolzen und eine umhüllende rohrförmige Hülse umfasst, in welche der Bolzen eingebracht ist, und dass das proximale Ende des inneren Bolzen und das proximale Ende der Hülse beide in der inneren Aussparung (35) des axialen Sperrelements (20) untergebracht sind.

## Revendications

1. Dispositif d'ostéosynthèse d'un os long (11) d'une jambe, comprenant une broche intramédullaire (12, 112) adaptée pour être insérée dans un orifice (14) formé dans le canal médullaire de l'os (11) entre un fond et une entrée (16), et comprenant un corps rigide et allongé (13, 113), qui s'étend le long d'un axe prédéterminé (X) entre une extrémité distale (18a, 118a) adaptée pour faire face au fond de l'orifice (14), et une extrémité proximale opposée (18b, 118b) adaptée pour faire face à l'entrée (16) de l'orifice (14), une pluralité d'éléments de verrouillage (15, 115) insérés dans des orifices transversaux correspondants (17, 117) formés dans le corps allongé (13, 113) de la broche (12, 112), et un élément d'arrêt axial (20) adapté pour être retenu à l'os à l'entrée (16) de l'orifice (14) et constituant un aboutement pour l'extrémité proximale (18b, 118b) de la broche (12, 112) ; lesdits éléments de verrouillage étant des éléments à mémoire de forme (115), **caractérisé en ce que** l'élément d'arrêt axial (20) est un corps ayant sensiblement une forme de coupe, qui est défini par un manteau (22), un fond (21) formant un aboutement d'arrêt pour la broche (12), et un bord libre (25), qui forme une cavité intérieure (35), où le manteau (22) du corps en forme de coupe porte un filetage extérieur (24) approprié pour être vissé dans l'os ; le manteau (22) du corps en forme de coupe comprenant une partie cylindrique (23), occupant une partie dominante du corps en forme de coupe commençant à partir du fond (21) et portant ledit filetage extérieur (24) et une partie conique lisse (30) avec une fonction d'auto-pénétration dans l'os, occupant une partie restante vers le bord libre (25) du corps en forme de coupe.

2. Dispositif d'ostéosynthèse selon la revendication 1, **caractérisé en ce que** le corps en forme de coupe possède une hauteur prédéterminée (L) et un diamètre extérieur (D), où la hauteur (L) a une valeur sensiblement égale à ou supérieure au diamètre extérieur (D).

3. Dispositif d'ostéosynthèse selon la revendication 1, **caractérisé en ce que** la cavité intérieure (35) occupe une partie dominante du corps en forme de coupe.

4. Dispositif d'ostéosynthèse selon la revendication 1, **caractérisé en ce que** l'élément d'arrêt axial (20) comprend un orifice traversant (41) pour loger un fil de guidage.

5. Dispositif d'ostéosynthèse selon la revendication 1, **caractérisé en ce qu'**il comprend trois éléments de verrouillage (115) dans une partie de la broche faisant face à l'extrémité proximale (118b).

6. Dispositif d'ostéosynthèse selon la revendication 1, **caractérisé en ce que** les éléments de verrouillage à mémoire de forme (115) sont des inserts structuralement indépendants du corps allongé (113).

7. Dispositif d'ostéosynthèse selon la revendication 1, **caractérisé en ce que** les éléments de verrouillage à mémoire de forme (115) ont une forme de fourche.

8. Dispositif d'ostéosynthèse selon la revendication 1, **caractérisé en ce que** les éléments de verrouillage à mémoire de forme (115) sont agencés de manière décalée les uns par rapport aux autres le long du corps allongé (113).

9. Dispositif d'ostéosynthèse selon la revendication 1, **caractérisé en ce que** le corps allongé (113) comprend une tige intérieure et une chemise d'enveloppe tubulaire, où la tige est insérée, et **en ce que** l'extrémité proximale de la tige intérieure et l'extrémité proximale de la chemise sont toutes les deux logées dans la cavité intérieure (35) de l'élément d'arrêt axial (20).
